(19) Europäisches Patentamt
European Patent Office
Office européen des brevets

(11) **EP 3 697 383 B1**

(12) **EUROPEAN PATENT SPECIFICATION**

(45) Date of publication and mention
of the grant of the patent:
**10.11.2021  Bulletin 2021/45**

(21) Application number: **18786348.5**

(22) Date of filing: **18.10.2018**

(51) Int Cl.:
*A61K 9/08* *(2006.01)*     *A61K 9/10* *(2006.01)*
*A61K 9/16* *(2006.01)*     *A61K 45/06* *(2006.01)*
*A61K 47/26* *(2006.01)*     *A61K 47/46* *(2006.01)*

(86) International application number:
**PCT/EP2018/078477**

(87) International publication number:
**WO 2019/077015 (25.04.2019 Gazette 2019/17)**

(54) **COMPOSITION FOR TREATING CONSTIPATION**

ZUSAMMENSETZUNG ZUR BEHANDLUNG VON VERSTOPFUNG

COMPOSITION DE TRAITEMENT DE LA CONSTIPATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.10.2017  IT 201700117749**

(43) Date of publication of application:
**26.08.2020  Bulletin 2020/35**

(73) Proprietor: **Neilos S.r.l.**
**80063 Piano di Sorrento (NA) (IT)**

(72) Inventor: **DI MAIO, Umberto**
**80063 Piano di Sorrento (NA) (IT)**

(74) Representative: **Longoni, Alessandra
AL & Partners Srl
Via C. Colombo ang. Via Appiani
(Corte del Cotone)
20831 Seregno (MB) (IT)**

(56) References cited:
**EP-A2- 2 014 181     EP-B1- 0 906 109**

**Description**

The present invention refers to a composition containing an association of lactitol, lactulose, the extract of at least one plant belonging to the genus *Plantago,* and inulin or fructooligosaccharides for the treatment of constipation. The composition is particularly effective thanks to the synergistic action of its components.

Background of the invention

Constipation is a common gastrointestinal disorder which affects about 28% of the Western world population. Epidemiological studies demonstrate that this problem is mainly encountered in elderly, children and women and it deeply interferes with the life quality of said subjects.

In recent years several scientific studies have focused on researching an adequate definition for the term constipation on the basis of some parameters such as faeces weight and consistency, time of colonic transit and evacuation difficulties.

The most important definitions refer to alterations of the above-mentioned parameters and constipation is mainly defined as a temporary or chronic condition wherein there is a delayed transit of faeces in the intestine entailing infrequent, difficult or incomplete evacuations. This condition can cause pain and abdominal distention as well as anorexia and vomit and other less specific symptoms such as headache, halitosis, confusion, etc.

The causes of constipation are mainly three: lifestyle (functional constipation), secondary condition of other diseases or induced by the use of some drugs. According to the most valid criteria, functional constipation is defined and diagnosed as the presence of symptoms for three months with an onset thereof for at least six months. The most specific diagnostic criteria are: straining during at least 25% of evacuations, hard and granulose faeces for at least 25% of evacuations, sensation of incomplete evacuation for at least 25% of evacuations, sensation of anal blockage or obstruction for at least 25% of evacuations, the use of manual maneuvers to facilitate at least 25% of evacuations, fewer than three evacuations per week and rarely loss of faeces. The physiopathology of functional constipation, however, remains still not well clear but it is believed to be mainly due to a slow colonic transit and/or pelvic floor dysfunctions. As regards the colonic transit, this slowing can be due to either excessive segmental contractions or a reduced contraction of distal colon.

As mentioned before, the constipation may also be due to the use of some drugs and in this case it is referred to as iatrogenic constipation. Primarily, opioid analgesics can induce alterations of the intestinal functionality through their action on the central nervous system and mainly on the opioid receptors present at enteric level. Also anticholinergic drugs can induce constipation by blocking the acetylcholine receptors present at intestinal level. Also tricyclic antidepressants show the same mechanism as anticholinergics in potentially causing constipation. Diuretics, by reducing the absorption of water during the formation of faeces or inhibiting the secretion, reduce intestinal motility and can lead to hypokalemia and constipation. Still other drugs that can cause constipation are antiparkinsonian agents, antihypertensives, MAOs inhibitors, antipsychotics, cholestyramine, etc.

Treatments usually performed in subjects suffering from this disorder vary according to the entity and cause of constipation. Initially, a conservative treatment comprising changes in diet and lifestyle, among which mostly a greater consumption of fibres, is preferred. In particular, in this regard reference is made to a consumption of about 30 g per day to ensure beneficial effects and prevent collateral events such as abdominal pain, flatulence and diarrhoea.

Another common practice is the so-called biofeedback which can be useful in effectively coordinating the pelvic floor distention and the anal sphincter relaxation in order to facilitate the passage of faeces.

When a simple variation in eating habits is not enough, pharmacological therapy is used which involves: bulk-forming laxatives and stool softeners, stimulant laxatives, osmotic laxatives, prokinetics and in the most serious cases enemas.

Bulk-forming laxatives have the same effect of fibres and include for example methylcellulose, psyllium and sterculia. These agents absorb water and increase the fecal mass promoting the frequency and consistency of faeces.

Docusate instead is the main agent used to soften faeces and its mechanism is linked to the ability to lower the surface tension of faeces facilitating the entry of water. Stimulant laxatives instead include plants containing anthraquinones (senna, cascara, aloe, frangula, rhubarb, etc.), diphenylamine derivatives (bisacodyl) and sodium picosulfate.

The use thereof is very common in both acute and chronic constipation and is linked to the ability to increase the intestinal motility and the secretion of water and electrolytes. Sodium picosulfate is mainly used for the preparation to endoscopy or other type of analysis.

Osmotic laxatives include magnesium and salts thereof, poorly absorbable sugars and polyethylene glycol-based preparations (PEG). These actives should represent the first line treatment to be followed in case the treatment with fibres or other changes in the lifestyle did not give the expected results. The mechanism of action thereof is linked to their ability to reach the colon in an unchanged manner establishing an osmotic gradient which increases fecal volume and peristalsis.

Prokinetic drugs are mainly cholinergic or serotonin 5-HT4 receptor agonists or of another type such as erythromycin, domperidone and metoclopramide.

The cholinergic agonists which are mostly recommended are bethanechol (25-50 mg 3 to 4 times a day reduces the constipation induced by tricyclic antidepressants) and neostigmine even if there are no significant scientific studies in support.

Among the 5-HT4 receptor agonists the main one is prucalopride which is recommended if the use of other types of laxatives has not brought about the desired effect. The use at a dosage between 1 and 2 mg increases the intestinal motility and decreases the time of colonic transit.

Now it has been surprisingly found that an association of lactitol, lactulose, an extract of one plant belonging to the genus *Plantago,* and inulin or fructooligosaccharides is particularly effective in the treatment of constipation thanks to the synergistic actions of its components.

*Detailed description of the invention*

Therefore, an object of the present invention is a composition containing an association of lactitol, lactulose, the extract of at least one plant belonging to the genus *Plantago,* and inulin or fructooligosaccharides useful for the treatment of constipation.

The components of the association according to the present invention are all known components, widely used in therapy.

EP 2 014 181, for example, discloses compositions comprising prebiotic and immunogenic ingredients such as galacto-oligosaccharides (GOSs), FOSs, inulin, isomalto-oligosaccharide, lactulose and polydextrose, useful for the treatment of gastroenteric disorders. However, it is completely silent about the usefulness of these compositions in patients suffering from constipation.

EP 0 906 109 B1 discloses in an example an aqueous laxative syrup comprising lactulose and lactose.

*Lactitol*

Lactitol, usually marketed in monohydrate form, is a disaccharide polyol composed of galactose and sorbitol units. Its chemical formula is $C_{12}H_{24}O_{11}$ and it is identified with the chemical name 4-O-β-D-galactopyranosyl-D-glucitol. Lactitol is obtained by catalytic hydrogenation of lactose from cow's milk affording an about 95% pure product. This polyol is approved by the European Union as food additive with the code E966 and used as synthetic sweetener.

Recently, the EFSA has received a positive opinion about the use of lactitol to obtain a normal intestinal functionality increasing the defecation frequency, faeces consistency and water content also reducing the time of intestinal transit.

This effect is surely justified by numerous clinical studies that were carried out on lactitol to evaluate a potential use thereof in the treatment of constipation.

Lactitol exerts these beneficial actions thanks to its intrinsic characteristics: in fact being a non-digestible sugar, firstly, it reaches in an unchanged manner the intestine wherein by osmotic effect it recalls huge amounts of water. Moreover, it is fermented and in this way it could increase the faeces volume and reduce the permanence time in the colon. It is known that said conditions are related to each other and facilitate defecation. From the fermentation of lactitol, acids (lactic acid) which contribute to maintain low the pH of bacterial flora, gas and short chain fatty acids (SCFAs), which could have a direct effect on the colonic motility, are formed.

As mentioned before, different clinical studies relative to lactitol administration in adults compared with both the placebo and lactulose or with other types of laxatives have been performed.

On the basis of a recent 2014 meta-analysis, it has emerged, from the analysis of the different clinical studies, that lactitol is well tolerated and is able to increase the evacuation frequency and the faeces consistency in patients affected by constipation. In particular, relative to the comparative studies with lactulose, one of the active ingredients most used and requested by patients with constipation, lactitol has shown a comparable efficacy and tolerability with a favourable trend for lactitol relatively to the evacuation frequency. Moreover, some studies report a better palatability and taste of products containing lactitol with respect to those containing lactulose, which could reduce some of the gastrointestinal symptoms and increase patient compliance. This effect is probably due to the low glycaemic index of lactitol which ensures an intake of only 2 calorie/g unlike other sugars. In fact, in marketed lactitol-based products, reference is made to the possibility of usage in diabetics as this disaccharide has no effects on glycaemia and insulinaemia.

Regarding the dosages and the safety of use, in general, lactitol-based formulations have proven to be effective and safe (without side effects such as diarrhoea or abdominal pain typically) at a dosage of 10 g per day. In fact several studies report the absence of diarrhoea at this dosage while a percentage between 5-20% of people involved in the studies were affected by diarrhoea, in case of using 20 g of active ingredient.

*Lactulose*

Lactulose is a disaccharide composed of fructose and galactose which usually is not present in milk but derives from heating processes; in fact, as the heating temperature increases a higher yield of this sugar is always obtained. It is commercially produced by reduction of lactose.

Lactulose is one of the active ingredients mostly used for the treatment of constipation which over the years has always maintained a good efficacy/safety profile.

This active is not absorbed in the gastrointestinal tract reaching in an unchanged form the colon where it performs its effect through osmotic action.

The lactulose action of increasing the intestinal transit by enhancing the evacuation has been demonstrated by some clinical studies that certify its efficacy and by the wide use over the years.

A first study carried out on 42 healthy volunteers and 24 subjects suffering from constipation assessed the effect of the administration of 20 and 40 g of lactulose per day. The results of the double-blind study have registered a significant increase of the frequency, volume and weight of faeces in both the study groups.

Another 1997 study assessed the effect of 10 g of lactulose with respect to the placebo to evaluate the oro-fecal transit in eight healthy individuals confirming an accelerating effect of the transit in the small intestine. Lactulose if compared to inulin has also proved to be effective in accelerating the transit when taken at a dosage of 10 g per day.

These data confirm the dated use of lactulose in the treatment of constipation. In fact, within the colon, lactulose is degraded into lactic acid, small amounts of acetic and formic acid through the action of beta-galactosidases of the bacterial flora. This process causes an increase in the osmotic pressure and a slight acidification of the colonic content which causes an increase of water content in the faeces which softens them making the defecation easier.

In case of particularly high dosages or use for prolonged periods of time, lactulose can causes light and transitional side effects such as flatulence, bloating and nausea. Despite these mild side effects the efficacy/safety ratio for this active is anyway high which is why lactulose represents a valid active ingredient to be used as a medicinal or nutraceutical product for the treatment of constipation.

*Extract of a plant belonging to the genus Plantago (Psyllium)*

With the term "psyllium" are commonly identified the plants or the seeds of different plants belonging to the genus *Plantago* and to the family of Plantaginaceae.

This genus comprises numerous plant species (for example *Plantago arenaria, Plantago indica, Plantago afra, Plantago lanceolata, Plantago major, Plantago media)* and in particular, those more recognised and cultivated are the species of *Plantago ovata* ("ispaghula") and *Plantago psyllium* to which reference is usually made when speaking of psyllium.

The functional part of the plant is represented by the seeds from which the tegument enveloping them is obtained which is commonly identified with the name of cuticle. The psyllium peel contains large amounts of hemicelluloses, while the seed is composed of 35% of soluble fibres and of 65% of insoluble polysaccharides. The psyllium is considered as a mucilaginous fibre because it is able to form a gel in aqueous solution. This ability is related to its role within the endosperm of the plant seeds, where it stores water preventing the drying.

To the psyllium are ascribed several properties potentially useful for treating constipation: said fibre is able to increase the weight of the fecal mass and favour the production of short chain fatty acids (SCFAs) particularly important for the intestine wellbeing. The increase of the fecal mass as well as of the faeces hydration is ascribable to the fibre ability to swallow in contact with biological fluids and to reach the intestinal caecum in an intact and highly polymerised form. Moreover, by partial fermentation of psyllium fibres SCFAs are produced among which acetate, propionate and butyrate. Butyric acid is particularly important for the enterocytes and from scientific studies it seems potentially valuable in cases of colon-rectal cancer and ulcerative colitis.

For these properties the psyllium has been evaluated as a valid remedy in cases of constipation, fecal incontinence, haemorrhoids, ulcerative colitis, to reduce appetite, for dyslipidaemias and for diabetes mellitus.

Its main use however remains relative to constipation about which several clinical studies have been carried out.

In particular, in a study carried out in 1997 by Voderholzer and collaborators the consumption of 15-30 g of psyllium per day assured an improvement or a resolution of the symptomatology in patients suffering from chronic constipation after a period of 6 weeks. The study highlights how the psyllium is particularly effective in case where there is not a clear pathological condition inducing constipation while it resulted less effective in patients with slow colonic transit or in patients with severe disorders such as rectocele, internal prolapses, rectal hyposensitivity or anismus.

Also in comparative studies psyllium has shown good results in terms of efficacy, for example in a comparative study with docusate sodium (100 mg 2 times per day) psyllium (5.1 g two times per day) has shown to be superior in patients with idiopathic chronic constipation. In particular the study has demonstarted that patients treated with a psyllium-based preparation showed a higher water content in the faeces and a general improvement of constipation symptoms which was much more significant by increasing the treatment time (treatment duration in the study 2 weeks).

In the light of the wide use in commercial products and on the basis of the good scientific documents, a plant extract belonging to the genus *Plantago* can be considered a valid component for the prevention, treatment or improvement of constipation.

*Inulin and fructooligosaccharides*

Inulin and fructooligosaccharides (FOSs) are compounds containing fructose chains bound to a terminal glucose wherein the fructose units are linked by $\beta(2\text{-}1)$ bonds and glucose is linked by an $\alpha(1\text{-}2)$ bond. From a chemical point of view FOSs and inulin differs solely in the polymerization degree which for the firsts is between 3 and 10 while inulin, which is a polymer, can contain up to 60.

Thanks to the presence of $\beta(2\text{-}1)$ type bonds, inulin and FOSs are not digestible by digestive enzymes in the small intestine and reach intact the colon wherein they are fermented forming SCFAs and gasses through the action of colon bacteria.

Said compounds are usually present in large amounts within onions, garlic, chicory, artichoke, leek, wheat and are probably the most studied and with the greatest usage for the intestinal well-being prebiotics. Inulin and FOSs reach the intestine in an unchanged manner after which they are fermented by the bacterial flora exerting their prebiotic effect. In fact, said oligosaccharides promote the proliferation of some species of bacteria present in the intestine, such as Bifidobacteria, with a consequent change in the composition of the intestinal flora with an increase of species producing positive effects and a reduction of potentially dangerous microorganisms.

The effects obtained by inulin on the gastrointestinal system include an increase in the defecation frequency, in particular in patients suffering from constipation.

In the literature there are several clinical studies which evaluated the effect of the daily consumption of inulin on the intestinal well-being (constipation and equilibrium of the bacterial flora).

For example, a recent 2017 study published on International Journal of Food Science and Nutrition by A. Micka and collaborators assessed the effect of inulin consumption (12 g per day) compared to placebo (maltodextrin at the same dosage) on the evacuation frequency, faeces consistency and gastrointestinal well-being of patients. From the results it is emerged a significant increase of the defecation frequency compared to placebo (4 compared to 3 evacuations per week), an improvement in faeces consistency and a positive impact on the life quality of patients.

Also on children between 2 and 5 years (n=22), the consumption of 4 g per day of a formulation containing inulin has shown to be promising for the treatment of functional constipation improving faeces consistency and defecation difficulties.

The consumption of inulin has shown to have beneficial effects also in healthy adults without constipation thanks to its prebiotic effect, ability to stimulate the immune system and decrease the stimulation of pro-inflammatory cytokines.

Besides their direct mild laxative action, inulin or FOSs represent important components within preparations intended for patients suffering from constipation for their prebiotic effect. This action is crucial to ensure a gastrointestinal and general well-being in patients.

In a preferred embodiment, the composition object of the present invention contain the association of lactitol, lactulose, the extract of at least one plant belonging to the genus *Plantago,* and inulin or fructooligosaccharides in admixture with a suitable acceptable carrier.

Suitable acceptable carriers are those commonly known to the man skilled in the art for the preparation of compositions for oral administration such as solutions, suspensions, powders or granulates, tablets, capsules, pellets. By way of non-limiting example, said acceptable carriers can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners. Specifically examples of diluents can be: magnesium carbonate, cellulose microcrystalline, starch, lactose, and sucrose; mainly used lubricants are magnesium stearate, stearic acid, and sodium stearyl fumarate. As glidants colloidal silica and magnesium silicate, as disintegrants the cross-linked polyvinylpyrrolidones and sodium starch glycolate, as solubilizing agents surfactants such as TWEEN or sodium lauryl sulphate, and as stabilizers all classes of preservatives (sorbic acid and derivatives, benzoic acid and derivatives, parabens), antioxidants (ascorbic acid and derivatives, tocopherol), and acidifying agents (phosphoric acid, tartaric acid) can be mentioned. Thickeners and gelling agents can be carrageenan, pectins and starches, coating agents include for example waxes and derivatives, anti-caking agents include for example calcium or magnesium carbonate, humectants include for example sorbitol and mannitol, sequestrants include for example EDTA and derivatives, sweeteners include for example aspartame and acesulfame potassium.

The composition object of the present invention is preferably a liquid or solid composition for oral use, even more preferably an aqueous solution, a suspension, a powder or granulate, a tablet, a capsule, a pellet.

The composition object of the present invention can be a medical device, a food supplement, a nutraceutical, dietetic and nutritional composition, a food product, a beverage, a nutraceutical product, a medicament, a medicated food, a pharmaceutical composition or a food for special medical purposes.

The composition object of the present invention contains lactitol, lactulose, the extract of at least one plant belonging to the genus *Plantago,* and inulin or fructooligosaccharides.

Lactitol is present in an amount between 400 mg and 30 g, preferably between 1 g and 20 g, still more preferably between 2 g and 10 g.

Lactulose is present in an amount between 400 mg and 30 g, preferably between 1 g and 10 g, still more preferably between 2 g and 5 g.

The extract of at least one plant belonging to the genus *Plantago* is preferably present in an amount between 50 mg and 10 g, preferably between 100 mg and 4000 mg, still more preferably between 200 mg and 2000 mg. In particular, the extract of at least one plant belonging to the genus *Plantago* is an extract of *Plantago ovata, Plantago psyllium, Plantago arenaria, Plantago indica, Plantago afra, Plantago lanceolata,*

*Plantago major* and/or *Plantago media.*

Inulin or fructooligosaccharides are present in an amount between 50 mg and 10 g, preferably between 100 mg and 4000 mg, still more preferably between 200 mg and 2000 mg.

The compositions object of the present invention are particularly effective in the treatment of constipation allowing to obtain at the same time the increase of evacuation frequency, increase of faeces weight and consistency and an improvement of intestinal wellbeing by prebiotic action thanks to the synergistic action of their components.

Therefore a further object of the present invention is a composition containing the association of lactitol, lactulose, an extract of a plant belonging to the genus *Plantago,* and inulin or fructooligosaccharides in admixture with a suitable acceptable carrier for use in the treatment of constipation.

Without being bound to a specific theory, the inventors are of the opinion that the synergistic effect of the association of lactitol, lactulose, the extract of at least one plant belonging to the genus *Plantago,* and inulin or fructooligosaccharides according to the present invention, derives from the following activities of the components of the association.

Lactitol and lactulose are non-absorbable disaccharides which reach in an unchanged form the colon where they are fermented by bacterial flora entailing a water recall through osmotic effect and causing an increase in the evacuation frequency.

The extract of at least one plant belonging to the genus *Plantago,* swelling in contact with biological fluids, is able to increase weight and to improve consistency of faeces. Moreover it favours the development of butyric acid particularly important for the enterocytes wellbeing.

Inulin and FOSs, besides having a mild laxative effect, are crucial for an adequate maintenance of intestinal flora bacteria.

The efficacy of the composition object of the present invention is evaluated with the following experimental protocol.

Constipation is induced through the administration to animals (mice and rats, preferably mouse) of compounds such as loperamide (3 mg/Kg) which binds to $\mu$, $\kappa$ and $\delta$ receptors leading to a reduction of muscle contractility at intestinal level and a reduction of secretions. Moreover said drug increases the absorption of water and reduces the contraction of sphincters reducing the evacuation frequency. Also other compounds that induce this type of effects or variation in diet (such as diets rich in fibres or with a high protein content) can anyway be used as experimental model of constipation.

After the induction of constipation, the compositions according to the present invention and the comparative compositions are orally administered to animals at established dosages comparing them with a control group normally administered with a saline solution or however an inert substance. To evaluate the potential laxative effect, the feces of animals are collected every day for a variable period of time. From the excreted feces, the number, the consistency in terms of weight and the water content (%), which is obtained from the ratio between wet feces weight minus dried feces weight divided by wet feces weight all multiplied by one hundred, are determined.

Another test that can be performed to assess the potential laxative action in terms of increase of intestinal motility is the study of the intestinal transit that is usually performed through the administration of coal or other similar compounds to animals after loperamide administration. Successively to constipation induction, animals are maintained without food in cages with wide meshes to facilitate feces fall.

After the administration (preferably after 30 minutes) of the compositions according to the present invention, or of the comparative compositions, animals are administered with a suspension (containing agents such as methylcellulose or preferably gum arabic) of coal (for example at 3-10%). After about 20-30 minutes the animals are sacrificed and the percentage of coal transit is obtained from the following equation:

total length of small intestine – distance covered by the coal/total length of small

intestine *100

Moreover, the synergistic effect of the composition can be highlighted evaluating the propulsion in the distal colon after six days from loperamide administration. Thirty minutes after samples administration (or the control and the vehicle), a glass pearl of 3 mm is inserted through the anus in the distal colon for 2 cm. For each animal the medium expulsion time (MET) of the glass pearl will be evaluated; an high MET value indicates an elevated reduction of the intestinal motility.

Using the above-described experimental protocols, the laxative action of the association of lactitol, lactulose, the extract of at least one plant belonging to the genus *Plantago* and inulin or fructooligosaccharides, according to the present invention, and of the individual components of the association is evaluate to verify the synergistic effect.

## Examples

By way of example are now provided some non-binding examples of daily doses of active components of the compositions object of the present invention.

Daily doses are meant to be administered in a suitable oral dosage form and divided in one or more dosage units.

EXAMPLE 1 (powder or granulate for oral use)

| Active ingredient | Amount for daily dose |
|---|---|
| Lactitol | 3 g |
| Lactulose | 3 g |
| Inulin | 800 mg |
| *Plantago ovata* | 200 mg |

EXAMPLE 2 (powder or granulate for oral use)

| Active ingredient | Amount for daily dose |
|---|---|
| Lactitol | 4 g |
| Lactulose | 4 g |
| Fructooligosaccharides | 500 mg |
| *Plantago psyllium* | 500 mg |

EXAMPLE 3 (solution or suspension for oral use)

| Active ingredient | Amount in 20 mL (daily dose) |
|---|---|
| Lactitol | 10 g |
| Lactulose | 4 g |
| Inulin | 800 mg |
| *Plantago ovata* | 200 mg |

Oral formulations are prepared by conventional techniques such as mixing

## Claims

1. A composition containing an association of lactitol, lactulose, the extract of at least one plant belonging to the genus *Plantago* and inulin or fructooligosaccharides in admixture with a suitable acceptable carrier.

2. A composition according to claim 1 wherein the extract of at least one plant belonging to the genus *Plantago* is an extract of *Plantago ovata, Plantago psyllium, Plantago arenaria, Plantago indica, Plantago afra, Plantago lanceolata, Plantago major* and/or *Plantago media.*

3. A composition according to claims 1 or 2 in the form of a liquid or solid composition for oral use.

4. A composition according to claim 3 in the form of aqueous solution, suspension, powder or granulate, capsule, tablet, pellet.

5. A composition according to any of the previous claims wherein lactitol is present in an amount between 400 mg and 30 g, preferably between 1 g and 10 g, still more preferably between 2 g and 10 g, lactulose is present in an amount between 400 mg and 30 g, preferably between 1 g and 10 g, still more preferably between 2 g and 5 g, the extract of at least one plant belonging to the genus *Plantago* is present in an amount between 50 mg and 10 g, preferably between 100 mg and 4000 mg, still more preferably between 200 mg and 2000 mg, and inulin or the fructooligosaccharides are present in an amount between 50 mg and 10 g, preferably between 100 mg and 4000 mg, still more preferably between 200 mg and 2000 mg.

6. Composition according to any of the previous claims wherein the composition is a medical device, a food supplement, a nutraceutical, dietetic and nutritional composition, a food product, a beverage, a nutraceutical product, a medicament, a medicated food, a pharmaceutical composition or a food for special medical purposes.

7. Composition according to any of the previous claims for use in the treatment of constipation.

**Patentansprüche**

1. Zusammensetzung, enthaltend eine Assoziation aus Lactitol, Lactulose, dem Extrakt mindestens einer Pflanze der Gattung *Plantago* und Inulin oder Fructooligosacchariden in Beimischung mit einem geeigneten annehmbaren Träger.

2. Zusammensetzung nach Anspruch 1, wobei der Extrakt mindestens einer Pflanze der Gattung *Plantago* ein Extrakt von *Plantago ovata, Plantago psyllium, Plantago arenaria, Plantago indica, Plantago afra, Plantago lanceolata, Plantago major* und/oder *Plantago media* ist.

3. Zusammensetzung nach Anspruch 1 oder 2 in der Form einer flüssigen oder festen Zusammensetzung zur oralen Anwendung.

4. Zusammensetzung nach Anspruch 3 in der Form einer wässrigen Lösung, einer Suspension, eines Pulvers oder Granulats, einer Kapsel, einer Tablette, eines Pellets.

5. Zusammensetzung nach einem der vorhergehenden Ansprüche, wobei Lactitol in einer Menge zwischen 400 mg und 30 g, bevorzugt zwischen 1 g und 10 g, noch bevorzugter zwischen 2 g und 10 g vorliegt, Lactulose in einer Menge zwischen 400 mg und 30 g, bevorzugt zwischen 1 g und 10 g, noch bevorzugter zwischen 2 g und 5 g vorliegt, der Extrakt mindestens einer Pflanze der Gattung *Plantago* in einer Menge zwischen 50 mg und 10 g, bevorzugt zwischen 100 mg und 4000 mg, noch bevorzugter zwischen 200 mg und 2000 mg vorliegt und Inulin oder die Fructooligosaccharide in einer Menge zwischen 50 mg und 10 g, bevorzugt zwischen 100 mg und 4000 mg, noch bevorzugter zwischen 200 mg und 2000 mg vorliegt/vorliegen.

6. Zusammensetzung nach einem der vorhergehenden Ansprüche, wobei die Zusammensetzung eine medizinische Vorrichtung, ein Nahrungsergänzungsmittel, eine nutrazeutische, diätetische und Ernährungszusammensetzung, ein Lebensmittelprodukt, ein Getränk, ein nutrazeutisches Produkt, ein Medikament, ein medizinisches Lebensmittel, eine pharmazeutische Zusammensetzung oder ein Lebensmittel für spezielle medizinische Zwecke ist.

7. Zusammensetzung nach einem der vorhergehenden Ansprüche zur Verwendung bei der Behandlung von Verstopfung.

**Revendications**

1. Composition contenant une association de lactitol, de lactulose, d'extrait d'au moins une plante appartenant au genre *Plantago* et d'inuline ou de fructooligosaccharides en mélange avec un vecteur acceptable approprié.

**2.** Composition selon la revendication 1, ledit extrait d'au moins une plante appartenant au genre *Plantago* étant un extrait de *Plantago ovata, Plantago psyllium, Plantago arenaria, Plantago indica, Plantago afra, Plantago lanceolata, Plantago major* et/ou *Plantago media*.

**3.** Composition selon la revendication 1 ou 2 sous forme de composition liquide ou solide à usage oral.

**4.** Composition selon la revendication 3 sous forme de solution aqueuse, suspension, poudre ou granulé, capsule, comprimé, pastille.

**5.** Composition selon l'une quelconque des revendications précédentes, ledit lactitol étant présent en une quantité comprise entre 400 mg et 30 g, de préférence entre 1 g et 10 g, encore plus préférablement entre 2 g et 10 g, ledit lactulose étant présent en une quantité comprise entre 400 mg et 30 g, de préférence entre 1 g et 10 g, encore plus préférablement entre 2 g et 5 g, ledit extrait d'au moins une plante appartenant au genre *Plantago* étant présent en une quantité comprise entre 50 mg et 10 g, de préférence entre 100 mg et 4000 mg, encore plus préférablement entre 200 mg et 2000 mg, et ladite inuline ou lesdits fructooligosaccharides étant présents en une quantité comprise entre 50 mg et 10 g, de préférence entre 100 mg et 4000 mg, encore plus préférablement entre 200 mg et 2000 mg.

**6.** Composition selon l'une quelconque des revendications précédentes, ladite composition étant un dispositif médical, un complément alimentaire, une composition nutraceutique, diététique et nutritionnelle, un produit alimentaire, une boisson, un produit nutraceutique, un médicament, un aliment médicamenteux, une composition pharmaceutique ou un aliment destiné à des fins médicales spéciales.

**7.** Composition selon l'une quelconque des revendications précédentes pour une utilisation dans le traitement de la constipation.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2014181 A

- EP 0906109 B1

**Non-patent literature cited in the description**

- **A. MICKA ; COLLABORATORS.** *International Journal of Food Science and Nutrition*